# EUROPÄISCHE PATENTSCHRIFT

(11) **EP 2 931 703 B1**
(45) Veröffentlichungstag und Bekanntmachung des Hinweises auf die Patenterteilung: **28.11.2018**
(21) Anmeldenummer: 13805850.8
(22) Anmeldetag: 12.12.2013
(51) Int. Cl.: C07C 319/06, C07C 319/22, C07C 319/24, C07C 323/09, C07C 323/34, C07C 323/37

(54) **VERFAHREN ZUR HERSTELLUNG VON BIS(3-AMINOPHENYL)-DISULFIDEN UND 3-AMINOTHIOLEN**
METHOD FOR PRODUCING BIS (3-AMINOPHENYL) DISULFIDES AND 3-AMINOTHIOLS
PROCÉDÉ DE FABRICATION DE BIS(3-AMINOPHÉNYLE)-DISULFIDES ET DE 3-AMINOTHIOLS

(30) Priorität: 12.12.2012 EP 12196615
(43) Veröffentlichungstag der Anmeldung: 21.10.2015
(73) Patentinhaber: Bayer CropScience AG, 40789 Monheim am Rhein (DE)
(72) Erfinder: HIMMLER, Thomas, 51519 Odenthal (DE); JOHANNA HAHN, Julia, 40591 Düsseldorf (DE)
(74) Vertreter: BIP Patents
(86) Internationale Anmeldenummer: PCT/EP2013/076301
(87) Internationale Veröffentlichungsnummer: WO 2014/090913

(56) Entgegenhaltungen:
- EP-A1- 0 093 363
- EP-A1- 0 416 361
- EP-A1- 0 517 924
- EP-A1- 0 903 340
- EP-A1- 1 803 712
- EP-A1- 2 143 724
- EP-A1- 2 226 312
- EP-A2- 0 347 370
- WO-A1-95/15954
- WO-A1-2007/066844
- WO-A1-2010/031813
- WO-A1-2011/083487
- WO-A1-2011/141568
- WO-A2-03/060098
- DD-A1- 149 363
- US-A- 2 986 581
- DATABASE CA [Online] CHEMICAL ABSTRACTS SERVICE, COLUMBUS, OHIO, US; 1990, SATO, JUN ET AL: "Preparation of bis(4-fluoro-2-chlorophenyl) disulfide derivatives as intermediates for herbicides", XP002691858, gefunden im STN Database accession no. 1991:101337 -& JP 2 221254 A (JAPAN) 4. September 1990 (1990-09-04)
- DATABASE CA [Online] CHEMICAL ABSTRACTS SERVICE, COLUMBUS, OHIO, US; 1996, WATANABE, JUNICHI ET AL: "Preparation of diphenyl disulfides as intermediates for agrochemical microbicides", XP002691857, gefunden im STN Database accession no. 1996:618177 -& JP 8 198842 A (NISSAN CHEMICAL IND LTD, JAPAN) 6. August 1996 (1996-08-06)
- DATABASE CA [Online] CHEMICAL ABSTRACTS SERVICE, COLUMBUS, OHIO, US; 2011, ABE, TETSUYA ET AL: "(+)-1-[2,4-Dimethyl-5-(2,2,2-trifluoroeth ylsulfinyl)phenyl]-3- (trifluoromethyl)-1H-1,2,4-triazole, its preparation by optical resolution of racemate, and pesticides containing it", XP002691859, gefunden im STN Database accession no. 2011:1404867 -& JP 2011 219419 A (KUMIAI CHEMICAL INDUSTRY CO., LTD., JAPAN; IHARA CHEMICAL INDUSTRY CO.) 4. November 2011 (2011-11-04)
- PILGRAM, KURT ET AL: "Reaction of cyclic phosphoramidites with disulfides. I. A novel synthesis of phosphoramidothioates", JOURNAL OF ORGANIC CHEMISTRY, 29(7), 1844 -7 CODEN: JOCEAH; ISSN: 0022-3263, 1964, XP002691860,
- PILGRAM, I. K. ET AL: "Reaction of nitroaryl thiocyanates with trialkyl phosphites", TETRAHEDRON, 20(2), 177 -87 CODEN: TETRAB; ISSN: 0040-4020, 1964, XP002691861,
- TOMITA, MASAO ET AL: "Antibacterial activity of some organic compounds in vitro. V. Antibacterial activity of diphenyl ethers and related compounds on Mycobacterium tuberculosis, Staphylococcus aureus, and Escherichia coli", YAKUGAKU ZASSHI, 72, 478 -82 CODEN: YKKZAJ; ISSN: 0031-6903, 1952, XP002691862,
- SIPYAGIN, ALEXEY M. ET AL: "Preparation of the first ortho-substituted pentafluorosulfanylbenzenes", JOURNAL OF FLUORINE CHEMISTRY , 112(2), 287-295 CODEN: JFLCAR; ISSN: 0022-1139, 2001, XP004312726,
- CHILD, REGINALD: "Sulfonation of o-anisidine and aceto-o-aniside", JOURNAL OF THE CHEMICAL SOCIETY 715-20 CODEN: JCSOA9; ISSN: 0368-1769, 1932, XP002691864,
- ZINCKE, TH. ET AL: "4,2-Aminotolylmercaptan", BERICHTE DER DEUTSCHEN CHEMISCHEN GESELLSCHAFT , 45, 1495-1511 CODEN: BDCGAS; ISSN: 0365-9496, 1912, XP002691865,
- HANSCH, CORWIN ET AL: "Catalytic synthesis of heterocycles. VIII. Dehydrocyclization of o-ethylbenzenethiols to thianaphthenes", JOURNAL OF ORGANIC CHEMISTRY , 21, 265-70 CODEN: JOCEAH; ISSN: 0022-3263, 1956, XP002691866,
- SRISOOK, EKARUTH ET AL: "The syntheses of 3-substituted 4-(pyridin-2-ylthio)indoles via Leimgruber-Batcho indole synthesis", BULLETIN OF THE KOREAN CHEMICAL SOCIETY , 25(6), 895-899 CODEN: BKCSDE; ISSN: 0253-2964, 2004, XP002721147, DOI: 10.5012/BKCS.2004.25.6.895
- EDWARD F. ELSLAGER ET AL: "Notes - Synthetic Schistosomicides. I. A Diphenylsulfide Analog of Miracil D", THE JOURNAL OF ORGANIC CHEMISTRY, Bd. 21, Nr. 12, 1. Dezember 1956 (1956-12-01), Seiten 1528-1529, XP055105480, ISSN: 0022-3263, DOI: 10.1021/jo01118a613
- BELLALE, EKNATH V. ET AL: "A simple, fast and chemoselective method for the preparation of arylthiols", SYNTHESIS , (19), 3211-3213 CODEN: SYNTBF; ISSN: 0039-7881, 2009, XP002721148, DOI: 10.1055/S-0029-1216955
- DATABASE REGISTRY [Online] CHEMICAL ABSTRACTS SERVICE, COLUMBUS, OHIO, US; 15. Juni 2012 (2012-06-15), XP002721149, Database accession no. 1378587-61-1
- DATABASE CA [Online] CHEMICAL ABSTRACTS SERVICE, COLUMBUS, OHIO, US; NISHIMURA, TOSHIHIRO ET AL: "Preparation of novel catechol derivatives as COMT inhibitors for treatment of Parkinson's disease", XP002721150, gefunden im STN Database accession no. 2008:1532384 -& JP 2008 308493 A (KISSEI PHARMACEUTICAL) 25. Dezember 2008 (2008-12-25)
- DATABASE CA [Online] CHEMICAL ABSTRACTS SERVICE, COLUMBUS, OHIO, US; NAGATA, TOSHIHIRO ET AL: "Preparation of S-(5-amino-2-chloro-4-fluorobenzene)thioac etic acid methyl ester and its intermediate", XP002721151, gefunden im STN Database accession no. 1992:530931 -& JP 4 108771 A (IHARA CHEMICAL KOGYO K. K., JAPAN) 9. April 1992 (1992-04-09)
- WANG, YAN ET AL: "Synthesis of "donor-bridge-acceptor" triad compounds containing the aromatic sulfur bridges", DYES AND PIGMENTS , VOLUME DATE 2000, 44(2), 93-100 CODEN: DYPIDX; ISSN: 0143-7208, 1999, XP002721152, DOI: 10.1016/S0143-7208(99)00076-5

## Beschreibung

Die vorliegende Erfindung betrifft ein neues Verfahren zur Herstellung von Bis(3-aminophenyl)-disulfiden der allgemeinen Formel (I) und 3-Aminothiolen der allgemeinen Formel (II) 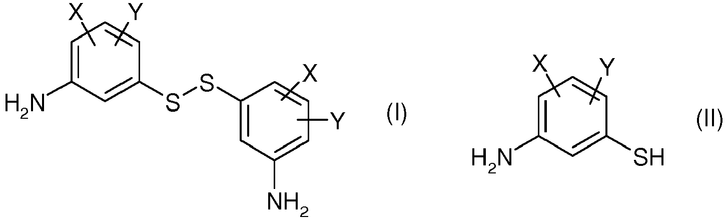 in welchen die Reste
X und Y unabhängig voneinander für Wasserstoff, linear oder verzweigtes (C₁-C₄)-Alkyl, linear oder verzweigtes (C₁-C₄)-Alkoxy, Halogen oder Amino stehen.

Bevorzugt stehen die Reste
X und Y unabhängig voneinander für Wasserstoff, linear oder verzweigtes (C₁-C₄)-Alkyl, Halogen oder Amino.

Besonders bevorzugt stehen die Reste
X und Y unabhängig voneinander für Wasserstoff, Methyl und Ethyl, Fluor und Chlor.

Bis(3-aminophenyl)-disulfide der allgemeinen Formel (I) und 3-Aminothiole der allgemeinen Formel (II) sind wichtige Zwischenprodukte zur Herstellung von Agrowirkstoffen und pharmazeutischen Wirkstoffen.

Die Herstellung von 3-Aminothiolen der allgemeinen Formel (II) ist bereits bekannt. So ist es beispielsweise möglich, 3-Nitrophenyl-sulfochloride der allgemeinen Formel (III) 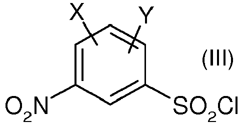 in der die Reste X und Y die oben angegebene Bedeutung haben,
in die 3-Aminothiole der allgemeinen Formel (II) zu überführen, indem man sie mit einem großen Überschuss eines Metalls wie Zink (EP 2 226 312) oder Zinn (J. Org. Chem. 21 (1956), 265-70) oder mit Zinn(II)-chlorid (Bioorganic & Medicinal Chemistry Letters 20 (2010) 1749-1751) in Gegenwart von Salzsäure und einem organischen Lösungsmittel umsetzt.

Bei diesen Reaktionen, bei denen sowohl die Nitro- als auch die Chlorsulfonylgruppe in einem Schritt reduziert werden, fallen große Mengen anorganischer Salze an, die aufwendig entsorgt werden müssen. Dies gilt insbesondere für umweltbelastende Metallsalze wie Zink- und Zinnchloride. Zudem sind die chemischen Ausbeuten bei dieser Reduktionsmethode nicht immer befriedigend.

Es sind auch mehrstufige Verfahren zur Herstellung aromatischer Disulfide bekannt geworden, in denen aromatische Sulfochloride zuerst mit Natriumhydrogensulfit zu den Natriumsalzen der betreffenden Sulfinsäuren umgesetzt werden, die dann mit Schwefeldioxid zu den Diphenyl-disulfiden reduziert werden (EP 687 671*;* WO 2007/066844). Diese Verfahren benötigen zum einen gasförmiges giftiges Schwefeldioxid, und liefern zum anderen nicht immer befriedigende Ausbeuten. Ebenfalls bekannt ist es, Diphenyl-disulfide ausgehend von aromatischen Sulfochloriden durch Reduktion mit Triphenylphosphin zu erhalten (Tetrahedron Letters 50 (2009) 7340-2)*.* Man kann eine solche Reduktion auch mittels Bromwasserstoff in einem Gemisch aus Essigsäure und Phenol durchführen (J. Fluor. Chem. 112 (2001) 287-95)*.* Diese Methoden führen aber nur zu unbefriedigenden Ausbeuten und hohen Abfallmengen. Am breitesten verwendet wird die Methode der Reduktion mit stöchiometrischen Mengen an Iodwasserstoff (J. Amer. Chem. Soc. 60 (1938) 2729-30*;* Organic Syntheses, Coll. Vol. 5(1973) 843*;* Vol. 40 (1960) 80*;* Synthesis 2003, 112-6). Diese Methode führt zwar zu hohen Ausbeuten, ist aber wegen der benötigten großen Menge an Iodwasserstoff und der Notwendigkeit der Entsorgung oder des Recyclings des entstehenden Iods sehr teuer und technisch aufwendig JP8198842 beschreibt die Herstellung von Diphenyldisulfiden, wobei Hydrazin als Reduktionsmittel verwendet wird.

Es bestand daher ein fortwährender Bedarf nach einer einfachen, ökonomisch vorteilhaften und ökologisch unbedenklichen Methode zur Reduktion von 3-Nitrophenyl-sulfochloriden der allgemeinen Formel (III) zu Bis(3-aminophenyl)-disulfiden der allgemeinen Formel (I) und 3-Aminothiolen der allgemeinen Formel (II).

Diese Aufgabe wurde nun überraschend durch die vorliegende Erfindung gelöst, die dadurch gekennzeichnet ist, dass 3-Nitrophenyl-sulfochloride der allgemeinen Formel (III) zunächst in einem ersten Schritt zu Bis(3-nitrophenyl)disulfiden der allgemeinen Formel (IV) 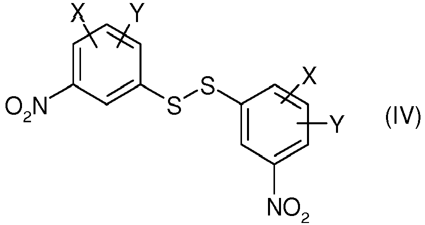 in der die Reste X und Y die oben angegebene Bedeutung haben, reduziert werden.

Diese Bis(3-nitrophenyl)disulfide der allgemeinen Formel (IV) werden anschließend in einem zweiten Schritt des erfindungsgemäßen Verfahrens zu den Bis(3-aminophenyl)-disulfiden der allgemeinen Formel (I) und den 3-Aminothiolen der allgemeinen Formel (II) reduziert.

Das erfindungsgemäße Verfahren wird also entsprechend dem Reaktionsschema A durchgeführt: 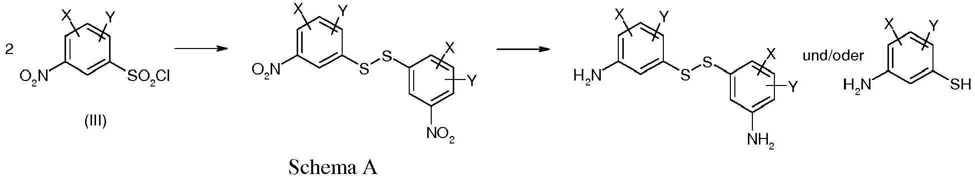 Obwohl das erfindungsgemäße Verfahren zweistufig durchgeführt wird, liefert es im Vergleich zu den bekannten einstufigen Methoden überraschenderweise die Bis(3-aminophenyl)disulfide der allgemeinen Formel (I) und die 3-Aminothiole der allgemeinen Formel (II) in besseren Ausbeuten oder höherer Reinheit, unter ökologisch und damit auch ökonomisch vorteilhaften Bedingungen.

Der erste Schritt des erfindungsgemäßen Verfahrens ist dadurch gekennzeichnet, dass die Reduktion von 3-Nitrophenyl-sulfochloriden der allgemeinen Formel (III) mit katalytischen Mengen an Iodid erfolgt, indem man die Reaktion in Gegenwart stöchiometrischer Mengen an Hypophosphoriger Säure (H₃PO₂) oder ihrer Salze durchführt.

Bevorzugt verwendet man die hypophosphorige Säure oder Natriumhypophosphit (NaH₂PO₂).

Die Menge an einzusetzender Hypophosphoriger Säure oder eines ihrer Salze beträgt 1 bis 2 Moläquivalente, bezogen auf das 3-Nitrophenyl-sulfochlorid der allgemeinen Formel (III). Bevorzugt setzt man 1,1 bis 1,8 Moläquivalente ein.

Als Katalysator können Iodwasserstoff, Metalliodide oder elementares Iod verwendet werden. Bevorzugt werden Metalliodide und elementares Iod verwendet; besonders bevorzugt sind Natrium- oder Kaliumiodid.

Die Menge an Katalysator kann in weiten Grenzen variiert werden. Für gewöhnlich nimmt man die kleinste Menge, die eine ausreichende Reaktionsgeschwindigkeit sicherstellt. Die Menge an Katalysator liegt dabei zwischen 0,1 und 20 Molprozent, bezogen auf das 3-Nitrophenyl-sulfochlorid der allgemeinen Formel (III). Bevorzugt setzt man 1 bis 20 Molprozent ein.

Als Lösungsmittel für den ersten Schritt des erfindungsgemäßen Verfahrens kommen prinzipiell Wasser und alle organischen Lösungsmittel in Frage, in denen die Reaktanden eine ausreichende Löslichkeit aufweisen. Als solche organischen Lösungsmittel seien beispielhaft genannt: Alkohole wie Methanol, Ethanol, Propanol, Isopropanol, Butanol, Isobutanol, Tertiärbutanol, Ethylenglykol; Ketone wie Aceton, Methyl-ethyl-keton, Methyl-isobutylketon; Ester wie Methylacetat und Ethylacetat; Ether wie Tetrahydrofuran, 2-Methyl-tetrahydrofuran, Dioxan, Methyl-tertiärbutyl-ether; Amide wie N,N-Dimethylformamid und N,N-Dimethylacetamid; Nitrile wie Acetonitril und Butyronitril; Carbonsäuren wie Ameisensäure, Essigsäure und Propionsäure. Es ist auch möglich, Mischungen dieser organischen Lösungsmittel untereinander oder mit Wasser zu verwenden.

Bevorzugt verwendet man Wasser, Methanol, Ethanol, Propanol, Isopropanol, Ethylenglykol, Aceton, Tetrahydrofuran, N,N-Dimethylformamid, Acetonitril, Butyronitril, Essigsäure, Propionsäure und Gemische dieser Lösungsmittel.

Besonders bevorzugt verwendet man Wasser, Methanol, Ethanol, Aceton, Essigsäure und Gemische dieser Lösungsmittel. Ganz besonders bevorzugt ist Essigsäure.

Die Temperatur im ersten Schritt des erfindungsgemäßen Verfahrens beträgt zwischen 0 und 150°C, bevorzugt zwischen 20 und 120°C.

Der erste Schritt des erfindungsgemäßen Verfahrens kann grundsätzlich auch unter vermindertem oder erhöhtem Druck durchgeführt werden.

Eine besonders bevorzugte Ausführungsform des erfindungsgemäßen Verfahrens ist dadurch gekennzeichnet, dass in Verfahrensschritt (A) Nitrophenyl-sulfochloride der allgemeinen Formel (III), in der die Reste X und Y die oben angegebene Bedeutung haben, mit katalytischen Mengen Iodid in Gegenwart stöchiometrischer Mengen an Hypophosphoriger Säure (H₃PO₂) oder ihrer Salze in einem organischen Lösungsmittel in Gegenwart von Wasser zu Bis(nitrophenyl)-disulfiden der allgemeinen Formel (IV) reduziert werden. In dieser Ausführungsform wird folglich der erste Schritt des erfindungsgemäßen Verfahrens in einem organischen Lösungsmittel in Gegenwart von Wasser durchgeführt.

Bei der Reduktion von Nitrophenyl-sulfochloriden der allgemeinen Formel (III) zu Bis(nitrophenyl)-disulfiden der allgemeinen Formel (IV) besteht die Möglichkeit einer Überreduktion zu Nitrophenyl-thiolen der allgemeinen Formel (V).

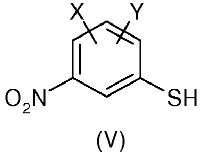

In einem solchen Fall werden dementsprechend dann Gemische bestehend aus den Bis(nitrophenyl)-disulfiden der allgemeinen Formel (IV) und den Nitrophenyl-thiolen der allgemeinen Formel (V) erhalten. Dies ist nicht nur hinsichtlich der analytischen Quantifizierung dieser Gemische nachteilig, sondern insbesondere auch dadurch, dass die Nitrophenyl-thiole der allgemeinen Formel (V), in der mindestens einer der Reste X und Y für Halogen steht, mit sich selbst bzw. mit den Bis(nitrophenyl)-disulfiden der allgemeinen Formel (IV), in der mindestens einer der Reste X und Y für Halogen steht, Kondensationsreaktionen unter Abspaltung von Halogenwasserstoff eingehen und durch die so gebildeten Nebenkomponenten die Produkte der Reduktion der Nitrophenyl-sulfochloride der allgemeinen Formel (III) zu Bis(nitrophenyl)-disulfiden der allgemeinen Formel (IV) verunreinigen und die erzielbare Ausbeute noch reduzieren können. Dies ist insbesondere dann der Fall, wenn die Reduktion der Nitrophenyl-sulfochloride der allgemeinen Formel (III) bei relativ hohen Konzentrationen der Nitrophenyl-sulfochloride durchgeführt wird, was aus ökonomischer und ökologischer Sicht zur Erzielung möglichst hoher Raum-Zeit-Ausbeuten außerordentlich wünschenswert ist. Die Bildung der unerwünschten Kondensationsprodukte der allgemeinen Formeln (VI) und (VII), in denen die Reste X und Y die oben angegebenen Bedeutungen haben, soll mit dem folgenden Schema verdeutlicht werden: 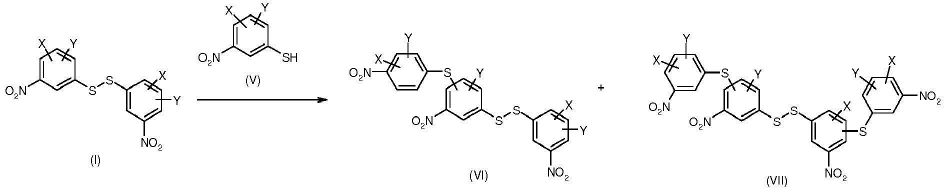 Die Gegenwart von Wasser bewirkt diesbezüglich eine selektive Reduktion der Nitrophenyl-sulfochloride der allgemeinen Formel (III) zu den Bis(nitrophenyl)-disulfiden der allgemeinen Formel (IV) ohne Bildung der Nitrophenyl-thiole der allgemeinen Formel (V) und damit auch ohne Bildung von unerwünschten Kondensationsprodukten der allgemeinen Formeln (VI) und (VII).

Bevorzugt verwendet man hypophosphorige Säure oder Natriumhypophosphit (NaH₂PO₂) in Gegenwart von Wasser. Dabei kann die hypophosphorige Säure beispielsweise in ihrer handelsüblichen Form als 50%ige Lösung in Wasser eingesetzt werden. Natriumhypophosphit (NaH₂PO₂) kann als wasserfreies Salz eingesetzt werden, wenn dem verwendeten Lösungsmittel Wasser zugesetzt wird. Alternativ ist es auch möglich, ein wasserfreies Lösungsmittel zu verwenden und das Natriumhypophosphit in Form seines Hydrates (NaH₂PO₂ x H₂O) einzusetzen.

Die Menge an Wasser in dieser besonders bevorzugten Ausführungsform des erfindungsgemäßen Verfahrens kann in weiten Grenzen variiert werden und hat im Prinzip kein oberes Limit, das allenfalls durch die Löslichkeit der Edukte im Gemisch des Lösungsmittels mit Wasser gegeben ist. Das untere Limit beträgt vorzugsweise 0,15 Gewichtsprozent, bezogen auf die Summe an organischem Lösungsmittel und Wasser.

In dieser besonders bevorzugten Ausführungsform des erfindungsgemäßen Verfahrens beträgt die Konzentration der Verbindung der allgemeinen Formel (III) vorzugsweise mindestens 0,6 mol/l und zunehmend bevorzugt 0,7 mol/l, 0,8 mol/l, 0,9 mol/l, 1,0 mol/l und 1,1 mol/l.

Im zweiten Schritt des erfindungsgemäßen Verfahrens werden die Bis(3-nitrophenyl)disulfide der allgemeinen Formel (IV) zu den Bis(3-aminophenyl)-disulfiden der Formel (I) und den 3-Aminothiolen der Formel (II) reduziert.

Dieser zweite Schritt des erfindungsgemäßen Verfahrens wird in der Weise durchgeführt, dass die Reduktion mit Wasserstoff in Gegenwart eines Metallkatalysators erfolgt, wobei als Metalle Nickel oder Cobalt verwendet werden.

Die Metalle können grundsätzlich in Form von Metallsalzen oder elementar eingesetzt werden. Werden die Metalle als solche verwendet, können sie entweder in reiner Form oder auf einem inerten Träger aufgebracht verwendet werden.

Nickel und Cobalt werden bevorzugt in Form der sogenannten Raney-Metalle eingesetzt.

Die Menge an Katalysator kann in weiten Grenzen variiert werden. Üblicherweise verwendet man 0,01 bis 50 Gewichtsprozent, bezogen auf das Bis(3-nitrophenyl)disulfid der allgemeinen Formel (IV). Bevorzugt verwendet man 1 bis 30 Gewichtsprozent von Raney-Nickel oder Raney-Cobalt.

Grundsätzlich können die heterogenen Katalysatoren nach der Reaktion auch zurückgewonnen und erneut eingesetzt werden.

Als Lösungsmittel für den zweiten Schritt des erfindungsgemäßen Verfahrens kommen grundsätzlich inerte organische Lösungsmittel in Frage. Beispielhaft seien hier genannt: Alkohole wie Methanol, Ethanol, Propanol, Isopropanol, Butanol, Isobutanol, Tertiärbutanol, Ethylenglykol; Ester wie Methylacetat und Ethylacetat; Ether wie Tetrahydrofuran, 2-Methyl-tetrahydrofuran, Dioxan, Methyl-tertiärbutyl-ether; Amide wie N,N-Dimethylformamid und N,N-Dimethylacetamid; Nitrile wie Acetonitril und Butyronitril; Carbonsäuren wie Ameisensäure, Essigsäure und Propionsäure. Es ist auch möglich, Mischungen dieser organischen Lösungsmittel untereinander zu verwenden.

Bevorzugt verwendet werden Methanol, Ethanol, Propanol, Isopropanol, Methylacetat, Ethylacetat, Tetrahydrofuran, 2-Methyl-tetrahydrofuran, Dioxan, Methyl-tertiärbutyl-ether.

Der Wasserstoffdruck im zweiten Schritt des erfindungsgemäßen Verfahrens liegt zwischen 1 und 150 bar, bevorzugt zwischen 5 und 100 bar.

Die Temperatur im zweiten Schritt des erfindungsgemäßen Verfahrens beträgt zwischen 20 und 200°C, bevorzugt zwischen 20 und 150°C.

Bei dieser katalytischen Reduktion der Bis(3-nitrophenyl)disulfide der allgemeinen Formel (IV) erhält man in aller Regel Gemische der Bis(3-aminophenyl)-disulfide der allgemeinen Formel (I) und der 3-Aminothiole der allgemeinen Formel (II). Die 3-Aminothiole der allgemeinen Formel (II) können nach bekannten Methoden der organischen Chemie in die Bis(3-aminophenyl)-disulfide der Formel (I) überführt werden. Für viele Zwecke, so zum Beispiel zur Herstellung von Alkylsulfanylbenzolen kann man jedoch sowohl Bis(3-aminophenyl)-disulfide der allgemeinen Formel (I) als auch 3-Aminothiole der allgemeinen Formel (II) mit gleich gutem Erfolg einsetzen (JCS Chem. Commun. 1991, 993-4*;* J. Fluor. Chem. 105 (2000) 41-44*;* Synthesis 2007, 85-91). Eine Aufreinigung der Gemische von Bis(3-aminophenyl)-disulfiden der allgemeinen Formel (I) und der 3-Aminothiole der allgemeinen Formel (II) ist in diesen Fällen also nicht notwendig.

Die Bis(3-aminophenyl)-disulfiden der allgemeinen Formel (I) und der 3-Aminothiole der allgemeinen Formel (II) dienen als Zwischenprodukte beispielsweise bei der Herstellung von insektizid, akarizid und nematizid wirksamen Phenylsulfoxiden, wie sie zum Beispiel aus der EP 1 803 712 und der WO 2011/006605 bekannt sind.

Ebenfalls Gegenstand der vorliegenden Erfindung sind neue Bis(3-nitrophenyl)disulfide der allgemeinen Formel (IV) 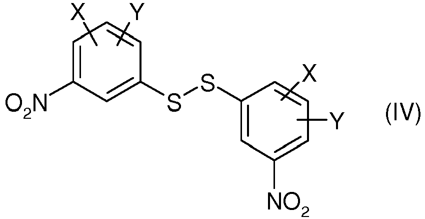 in der die Reste X und Y unabhängig voneinander für Methyl, Ethyl, Methoxy, Ethoxy und Fluor stehen.

Bevorzugt stehen die Reste X und Y unabhängig voneinander für Methyl und Fluor.

Ganz besonders bevorzugt sind folgende Verbindungen:
1,1'-Disulfandiylbis(4-fluor-2-methyl-5-nitrobenzol),
1,1'-Disulfandiylbis(2-fluor-4-methyl-5-nitrobenzol),
1,1'-Disulfandiylbis(4-fluor-2-methyl-3-nitrobenzol),
1-Fluor-4-[(4-fluor-2-methyl-5-nitrophenyl)disulfanyl]-3-methyl-2-nitrobenzol,
1-Fluor-4-[(2-fluor-4-methyl-5-nitrophenyl)disulfanyl]-5-methyl-2-nitrobenzol,
1-Fluor-4-[(2-fluor-4-methyl-5-nitrophenyl)disulfanyl]-3-methyl-2-nitrobenzol.

Ebenfalls Gegenstand der vorliegenden Erfindung sind neue Bis(3-aminophenyl)-disulfide der Formel (I) 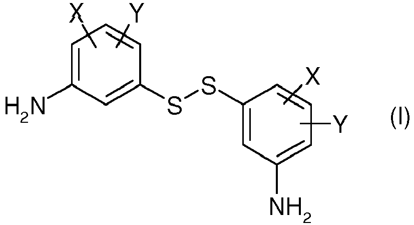 in der die Reste X und Y unabhängig voneinander für Methyl, Ethyl, Methoxy, Ethoxy und Fluor stehen.

Bevorzugt stehen die Reste X und Y unabhängig voneinander für Methyl und Fluor.
Ganz besonders bevorzugt sind folgende Verbindungen:
3,3'-Disulfandiylbis(6-fluor-4-methylanilin),
3,3'-Disulfandiylbis(4-fluor-6-methylanilin),
3,3'-Disulfandiylbis(2-fluor-6-methylanilin),
3-[(5-Amino-4-fluor-2-methylphenyl)disulfanyl]-6-fluor-2-methylanilin,
5-[(5-Amino-2-fluor-4-methylphenyl)disulfanyl]-2-fluor-4-methylanilin,
3-[(5-Amino-2-fluor-4-methylphenyl)disulfanyl]-6-fluor-2-methylanilin.

Ebenfalls Gegenstand der vorliegenden Erfindung ist die Verbindung 3,3'-Disulfandiylbis(4,6-dichloranilin).

Ebenfalls Gegenstand der vorliegenden Erfindung sind neue 3-Aminothiole der allgemeinen Formel (II) 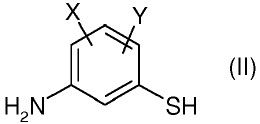 in der die Reste X und Y unabhängig voneinander für Wasserstoff, Methyl, Ethyl, Methoxy, Ethoxy, Fluor und Chlor stehen, wobei die Verbindungen ausgewählt sind aus
5-Amino-2,4-dichlorbenzolthiol, und
3-Amino-2-fluor-4-methylbenzolthiol.

Das erfindungsgemäße Verfahren soll durch die folgenden Beispiele veranschaulicht werden, ohne auf diese eingeschränkt zu sein.

### Beispiel 1

### 1,1'-Disulfanediylbis(4-fluor-2-methyl-5-nitrobenzol)

Zu einer Lösung von 50,7 g [200 mmol] 4-Fluor-2-methyl-5-nitrobenzolsulfonylchlorid in 350 ml Essigsäure gibt man 3,32 g [20 mmol] Kaliumiodid und erwärmt auf 40-45°C. Bei dieser Temperatur werden portionsweise innerhalb von etwa 50 Minuten 30,39 g [345 mmol] Natriumhypophosphit zugegeben. Man rührt 8 Stunden bei 40-45°C, kühlt auf Raumtemperatur ab und destilliert den größten Teil der Essigsäure ab. Der Rückstand wird mit 150 ml Wasser verrührt. Man saugt den ausgefallenen Feststoff ab, wäscht ihn mit Wasser und trocknet. Man erhält 36,75 g Feststoff einer Reinheit von 97,4%(w/w) (96,1% der Theorie).
¹H-NMR (600 MHz, CD₃CN): δ = 2,50 (s, 6H), 7,34 (d, J = 12,0 Hz, 2 H), 8,13 (d, J = 7,5 Hz, 2 H) ppm.
¹⁹F-NMR (566 MHz, CDCl₃): δ = -118,6 ppm.

### Beispiel 2

### 1,1'-Disulfanediylbis(4-fluor-2-methyl-5-nitrobenzol)

Zu einer Lösung von 12,7 g [50 mmol] 4-Fluor-2-methyl-5-nitrobenzolsulfonylchlorid in 100 ml Essigsäure gibt man 1,66 g [10 mmol] Kaliumiodid und 10 g [75.8 mmol] 50%ige wässrige Hypophosphorige Säure. Man erwärmt für 6 Stunden auf 60-95°C, kühlt auf Raumtemperatur ab und engt am Rotationsverdampfer ein.. Der Rückstand wird mit 100 ml Wasser aufgenommen und mit 100 ml Essigester extrahiert. Die organische Phase wird mit 20 ml halbkonzentrierter Natriumbisulfitlösung, 50 ml Wasser und 100 ml Natriumbicarbonatlösung gewaschen, getrocknet und eingeengt. Man erhält 8,8 g Feststoff in einer Reinheit von 92,1% (87% der Theorie).

### Beispiel 3

### 1,1'-Disulfanediylbis(2-fluor-4-methyl-5-nitrobenzol)

Analog der Vorgehensweise aus Beispiel 1.
¹⁹F-NMR (566 MHz, CDCl₃): δ = -101,6 ppm.

### Beispiel 4

### 1,1'-Disulfandiylbis(4-fluor-2-methyl-3-nitrobenzol)

Analog der Vorgehensweise aus Beispiel 1.
¹⁹F-NMR (566 MHz, CDCl₃): δ = -122 ppm.

### Beispiel 5

### 1,1'-Disulfandiylbis(2,4-dichlor-5-nitrobenzol)

Analog der Vorgehensweise aus Beispiel 1.
logP(HCOOH): 5,69; logP(neutral): 5,64
¹H-NMR (d-DMSO, 400MHz) δ = 8,33(s,2H), 8,21(s,2H) ppm.

### Beispiel 6

### 1-Fluor-4-[(2-fluor-4-methyl-5-nitrophenyl)disulfanyl]-5-methyl-2-nitrobenzol

Analog der Vorgehensweise aus Beispiel 1.
¹⁹F-NMR (566 MHz, CDCl₃): δ = -101.6 und -118,4 ppm.

### Beispiel 7

### 3,3'-Disulfanediylbis(6-fluor-4-methylanilin)

Man hydriert 1,96 g [5,27 mmol] 1,1'-Disulfanediylbis(4-fluor-2-methyl-5-nitrobenzol) in 50 ml Tetrahydrofuran an 0,4 g Raney-Cobalt (Actimet) 19 Stunden bei 65°C und 30 bar Wasserstoffdruck. Nach abfiltrieren des Katalysators wird das Lösungsmittel am Rotationsverdampfer abgezogen. Man erhält 1,7 g Feststoff, der nach HPLC-Analyse 81,3% 3,3'-Disulfandiylbis(6-fluor-4-methylanilin) und 13,3% 5-Amino-4-fluor-2-methylbenzolthiol enthält (97,5% der Theorie).
LC/MS: 5-Amino-4-fluor-2-methylbenzolthiol: m/e = 158 (MH⁺)
3,3'-Disulfandiylbis(6-fluor-4-methylanilin: m/e = 313 (MH⁺)
GC/MS(sil.): 5-Amino-4-fluor-2-methylbenzolthiol: m/e = 301 (M⁺, 2 x sil, 50%), 286 (< 5%), 181 (60%), 73 (100%).
3,3'-Disulfandiylbis(6-fluor-4-methylanilin: m/e = 456 (M⁺, 2 x sil, 100%), 441 (5%), 228 (100%), 73 (100%).
¹H-NMR (600 MHz, d-DMSO): δ = 2,16 (s, 6 H), 2,5 (m, 4 H), 6,9-7 (m, 4 H) ppm.
¹⁹F-NMR (566 MHz, CDCl₃): δ = - 134,5 ppm.

### Beispiel 8

### 3,3'-Disulfanediylbis(6-fluor-4-methylanilin)

Man hydriert 152 g [408 mmol] 1,1'-Disulfanediylbis(4-fluor-2-methyl-5-nitrobenzol) in 810 ml Tetrahydrofuran an 16 g Raney-Cobalt (Actimet) 19 Stunden bei 65°C und 30 bar Wasserstoffdruck. Nach abfiltrieren des Katalysators wird das Lösungsmittel am Rotationsverdampfer abgezogen. Man erhält 134,5 g Feststoff, der nach HPLC-Analyse 19,8% 3,3'-Disulfandiylbis(6-fluor-4-methylanilin) und 75,1% 5-Amino-4-fluor-2-methylbenzolthiol enthält (99,6% der Theorie).

### Beispiel 9

### 3,3'-Disulfanediylbis(6-fluor-4-methylanilin)

Man hydriert 2,91 g [7,8 mmol] 1,1'-Disulfanediylbis(4-fluor-2-methyl-5-nitrobenzol) in 35 ml Tetrahydrofuran an 0,3 g Raney-Nickel (A 4000) 19 Stunden bei 65°C und 30 bar Wasserstoffdruck. Nach abfiltrieren des Katalysators wird das Lösungsmittel am Rotationsverdampfer abgezogen. Man erhält 2,6 g Feststoff, der nach HPLC-Analyse 67,3% 3,3'-Disulfandiylbis(6-fluor-4-methylanilin) und 12,6% 5-Amino-4-fluor-2-methylbenzolthiol enthält (85% der Theorie).

### Beispiel 10

### 3,3'-Disulfanediylbis(6-fluor-4-methylanilin)

Man hydriert 34,2 g [91,8 mmol] 1,1'-Disulfanediylbis(4-fluor-2-methyl-5-nitrobenzol) in 180 ml Methyl-tertiärbutylether an 1,8 g Raney-Cobalt (Actimet) 19 Stunden bei 65°C und 30 bar Wasserstoffdruck. Nach abfiltrieren des Katalysators wird das Lösungsmittel am Rotationsverdampfer abgezogen. Man erhält 29,7 g Feststoff, der nach HPLC-Analyse 8,3% 3,3'-Disulfandiylbis(6-fluor-4-methylanilin) und 89,5% 5-Amino-4-fluor-2-methylbenzolthiol enthält (92% der Theorie).

### Beispiel 11

### 3,3'-Disulfanediylbis(6-fluor-4-methylanilin)

Man hydriert 34,2 g [91,8 mmol] 1,1'-Disulfanediylbis(4-fluor-2-methyl-5-nitrobenzol) in 180 ml Essigester an 1,8 g Raney-Cobalt (Actimet) 19 Stunden bei 65°C und 30 bar Wasserstoffdruck. Nach abfiltrieren des Katalysators wird das Lösungsmittel am Rotationsverdampfer abgezogen. Man erhält 30,1 g Feststoff, der nach HPLC-Analyse 8,9% 3,3'-Disulfandiylbis(6-fluor-4-methylanilin) und 89,3% 5-Amino-4-fluor-2-methylbenzolthiol enthält.

### Beispiel 12

### 3,3'-Disulfanediylbis(6-fluor-4-methylanilin)

Man hydriert 34,2 g [91,8 mmol] 1,1'-Disulfanediylbis(4-fluor-2-methyl-5-nitrobenzol) in 180 ml Isobutylalkohol an 1,8 g Raney-Cobalt (Actimet) 19 Stunden bei 65°C und 30 bar Wasserstoffdruck. Nach abfiltrieren des Katalysators wird das Lösungsmittel am Rotationsverdampfer abgezogen. Man erhält 30 g Feststoff, der nach HPLC-Analyse 16% 3,3'-Disulfandiylbis(6-fluor-4-methylanilin) und 80,7% 5-Amino-4-fluor-2-methylbenzolthiol enthält.

### Beispiel 13 (nicht erfindungsgemäß)

### 3,3'-Disulfanediylbis(6-fluor-4-methylanilin)

Man hydriert 0,97 g [2,6 mmol] 1,1'-Disulfanediylbis(4-fluor-2-methyl-5-nitrobenzol) in 9 ml Tetrahydrofuran an 44 mg 5% Pt/C (Evonik F 105 NC/W) 19 Stunden bei 65°C und 60 bar Wasserstoffdruck. Nach abfiltrieren des Katalysators wird das Lösungsmittel am Rotationsverdampfer abgezogen. Man erhält 0,87 g Feststoff, der nach HPLC-Analyse 8% 3,3'-Disulfandiylbis(6-fluor-4-methylanilin) und 88,3% 5-Amino-4-fluor-2-methylbenzolthiol enthält.

### Beispiel 14

### 3,3'-Disulfandiylbis(4,6-dichloranilin)

Analog der Vorgehensweise aus Beispiel 9.
logP(HCOOH): 5,14; logP(neutral): 4,95
¹H-NMR (d-DMSO, 400MHz) δ = 7,41(s,2H), 6,95(s,2H), 5,78 (breit, 4H) ppm.
GC-MS: EI-Masse (m/z): 386 (4 Cl) [M]⁺

Die nachfolgenden Beispiele und Vergleichsbeispiele betreffen Ausgestaltungen von Verfahrensschritt (A) des erfindungsgemäßen Verfahrens. Das vorzugsweise erhaltene Produkt ist daher ein Bis(3-nitrophenyl)disulfid der allgemeinen Formel (IV).

### Beispiel 15

### 1,1'-Disulfanediylbis(4-fluor-2-methyl-5-nitrobenzol)

Zu einer Lösung von 25,4 g [100 mmol] 4-Fluor-2-methyl-5-nitrobenzolsulfonylchlorid in 50 g (47,7 ml) Essigsäure gibt man 1,66 g [10 mmol] Kaliumiodid und erwärmt auf 60°C. Bei dieser Temperatur werden portionsweise innerhalb von etwa 100 Minuten 15,9 g [150 mmol] Natriumhypophosphit-Monohydrat zugegeben (entspricht 2,7 g Wasser bzw. 5,1 Gew.%). Man rührt 6 Stunden bei 60-62°C, kühlt auf 40°C ab, gibt 50 ml Wasser hinzu und rührt 30 Minuten bei 40°C. Anschließend wird auf 10°C abgekühlt, der ausgefallene Feststoff abfiltriert, mit 60 ml Eiswasser gewaschen und getrocknet. Man erhält 16,99 g Feststoff.
HPLC-Analyse: 92,9 Fl.% (IV), < 0,1 Fl.% (V); < 0,1 Fl.% (VI), < 0,1 Fl.% (VII)
Ausbeute: 85% der Theorie.
¹H-NMR (600 MHz, CD₃CN): δ = 2,50 (s, 6H), 7,34 (d, J = 12,0 Hz, 2 H), 8,13 (d, J = 7,5 Hz, 2 H) ppm.
¹⁹F-NMR (566 MHz, CDCl₃): δ = -118,6 ppm.

### Beispiel 16

### 1,1'-Disulfanediylbis(4-fluor-2-methyl-5-nitrobenzol)

Zu einer Lösung von 25,4 g [100 mmol] 4-Fluor-2-methyl-5-nitrobenzolsulfonylchlorid in 50 g (47,7 ml) Essigsäure und 0,54 g Wasser (entspricht 1,07 Gew.%) gibt man 1,66 g [10 mmol] Kaliumiodid und erwärmt auf 60°C. Bei dieser Temperatur werden portionsweise innerhalb von etwa 100 Minuten 13,2 g [150 mmol] Natriumhypophosphit mit einem Wassergehalt von etwa 0,4% zugegeben. Man rührt 6 Stunden bei 60-62°C, kühlt auf 40°C ab, gibt 50 ml Wasser hinzu und rührt 30 Minuten bei 40°C. Anschließend wird auf 10°C abgekühlt, der ausgefallene Feststoff abfiltriert, mit 60 ml Eiswasser gewaschen und getrocknet. Man erhält 17,34 g Feststoff.
HPLC-Analyse: 91,6 Fl.% (IV), < 0,1 Fl.% (V); < 0,1 Fl.% (VI), < 0,1 Fl.% (VII)
Ausbeute: 85% der Theorie.

### Vergleichsbeispiel 1

### 1,1'-Disulfanediylbis(4-fluor-2-methyl-5-nitrobenzol)

Zu einer Lösung von 25,4 g [100 mmol] 4-Fluor-2-methyl-5-nitrobenzolsulfonylchlorid in 50 g (47,7 ml) Essigsäure gibt man 1,66 g [10 mmol] Kaliumiodid und erwärmt auf 60°C. Bei dieser Temperatur werden portionsweise innerhalb von etwa 100 Minuten 13,2 g [150 mmol] wasserfreies Natriumhypophosphit mit einem Wassergehalt von etwa 0,4% zugegeben. Man rührt 6 Stunden bei 60-62°C, kühlt auf 40°C ab, gibt 50 ml Wasser hinzu und rührt 30 Minuten bei 40°C. Anschließend wird auf 10°C abgekühlt, der ausgefallene Feststoff abfiltriert, mit 60 ml Eiswasser gewaschen und getrocknet. Man erhält 17,14 g Feststoff.
HPLC-Analyse: 8,7 Fl.% (IV), 83,7 Fl.% (V); 2,2 Fl.% (VI), 0,3 Fl.% (VII)
Ausbeute: 8% der Theorie an (IV), 74,6% der Theorie an (V).

### Beispiel 17

### 1,1'-Disulfanediylbis(4-fluor-2-methyl-5-nitrobenzol)

Zu einer Lösung von 25,4 g [100 mmol] 4-Fluor-2-methyl-5-nitrobenzolsulfonylchlorid in 96,5 g (92 ml) Essigsäure und 3 g Wasser (entspricht 3,0 Gew.%) gibt man 1,66 g [10 mmol] Kaliumiodid und erwärmt auf 60°C. Bei dieser Temperatur werden portionsweise innerhalb von etwa 100 Minuten 13,2 g [150 mmol] wasserfreies Natriumhypophosphit zugegeben. Man rührt 5 Stunden bei 58-62°C, kühlt auf 40°C ab, gibt 50 ml Wasser hinzu und rührt 30 Minuten bei 40°C. Anschließend wird auf 10°C abgekühlt, der ausgefallene Feststoff abfiltriert, mit 60 ml Eiswasser gewaschen und getrocknet. Man erhält 16,54 g Feststoff.
HPLC-Analyse: 92,9 Fl.% (IV), < 0,1 Fl.% (V); < 0,1 Fl.% (VI), < 0,1 Fl.% (VII)
Ausbeute: 83% der Theorie.

### Beispiel 18

### 1,1'-Disulfanediylbis(4-fluor-2-methyl-5-nitrobenzol)

Man verfährt wie in Beispiel 17, setzt jedoch 98,1 g (93,5 ml) Essigsäure und 1,5 g Wasser (entspricht 1,5 Gew.%) ein. Man erhält 16,91 g Feststoff.
HPLC-Analyse: 92,1 Fl.% (IV), < 0,1 Fl.% (V); < 0,1 Fl.% (VI), < 0,1 Fl.% (VII)
Ausbeute: 84% der Theorie.

### Beispiel 19

### 1,1'-Disulfanediylbis(4-fluor-2-methyl-5-nitrobenzol)

Man verfährt wie in Beispiel 17, setzt jedoch 98,85 g (94,2 ml) Essigsäure und 0,75 g Wasser (entspricht 0,38 Gew.%) ein. Man erhält 17,31 g Feststoff.
HPLC-Analyse: 90,9 Fl.% (IV), < 0,1 Fl.% (V); < 0,1 Fl.% (VI), < 0,1 Fl.% (VII)
Ausbeute: 84% der Theorie.

### Beispiel 20

### 1,1'-Disulfanediylbis(4-fluor-2-methyl-5-nitrobenzol)

Man verfährt wie in Beispiel 17, setzt jedoch 99,2 g (94,6 ml) Essigsäure und 0,375 g Wasser (entspricht 0,38 Gew.%) ein. Man erhält 17,20 g Feststoff.
HPLC-Analyse: 92 Fl.% (IV), < 0,1 Fl.% (V); < 0,1 Fl.% (VI), < 0,1 Fl.% (VII)
Ausbeute: 86% der Theorie.

### Beispiel 21

### 1,1'-Disulfanediylbis(4-fluor-2-methyl-5-nitrobenzol)

Man verfährt wie in Beispiel 17, setzt jedoch 99,4 g (94,8 ml) Essigsäure und 0,188 g Wasser (entspricht 0,19 Gew.%) ein. Man erhält 16,97 g Feststoff.
HPLC-Analyse: 94,6 Fl.% (IV), < 0,1 Fl.% (V); < 0,1 Fl.% (VI), < 0,1 Fl.% (VII)
Ausbeute: 86% der Theorie.

### Vergleichsbeispiel 2

### 1,1'-Disulfanediylbis(4-fluor-2-methyl-5-nitrobenzol)

Man verfährt wie in Beispiel 17, setzt jedoch 99,5 g (94,9 ml) Essigsäure und 0,094 g Wasser (entspricht 0,09 Gew.%) ein. Man erhält 16,37 g Feststoff.
HPLC-Analyse: 37,8 Fl.% (IV), 49,5 Fl.% (V); 8,7 Fl.% (VI), 0,9 Fl.% (VII)
Ausbeute: 33,2% der Theorie an (IV), 43,3% der Theorie an (V).

### Vergleichsbeispiel 3

### 1,1'-Disulfanediylbis(4-fluor-2-methyl-5-nitrobenzol)

Man verfährt wie in Beispiel 17, setzt jedoch 100 g (95,3 ml) Essigsäure ohne Zusatz von Wasser ein. Man erhält 15,96 g Feststoff.
HPLC-Analyse: 5,0 Fl.% (IV), 89,3 Fl.% (V); 2,2 Fl.% (VI), 0,4 Fl.% (VII)
Ausbeute: 4,3% der Theorie an (IV), 75,8% der Theorie an (V).

## Patentansprüche

1. Verfahren zur Herstellung von Bis(3-aminophenyl)-disulfiden der allgemeinen Formel (I) und/oder 3-Aminothiolen der allgemeinen Formel (II) 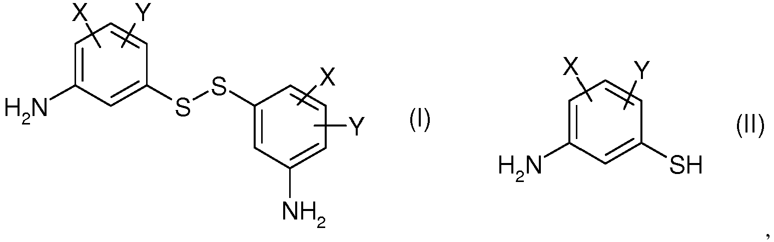 wobei die Reste X und Y unabhängig voneinander für Wasserstoff, linear oder verzweigtes (C₁-C₄)-Alkyl, linear oder verzweigtes (C₁-C₄)-Alkoxy, Halogen oder Amino stehen;
**dadurch gekennzeichnet, dass**
(A) 3-Nitrophenyl-sulfochloride der allgemeinen Formel (III) 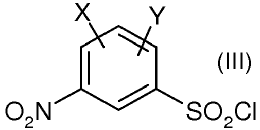 wobei die Reste X und Y die oben angegebenen Bedeutungen haben,
mit katalytischen Mengen Iodid in Gegenwart hypophosphoriger Säure (H₃PO₂) oder ihrer Salze reduziert werden zu Bis(3-nitrophenyl)disulfiden der allgemeinen Formel (IV) 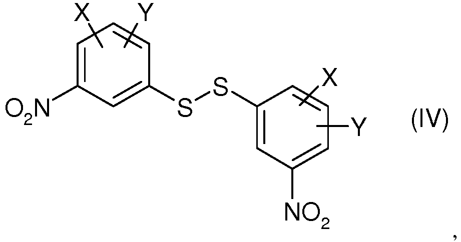 in der die Reste X und Y die oben angegebene Bedeutung haben,
(B) diese Verbindungen der allgemeinen Formel (IV) mit Wasserstoff in Gegenwart eines Metallkatalysators zu den Bis(3-aminophenyl)-disulfiden der allgemeinen Formel (I) und/oder 3-Aminothiolen der allgemeinen Formel (II) reduziert werden, wobei als Metalle Nickel oder Cobalt verwendet werden.

2. Verfahren gemäß Anspruch 1, **dadurch gekennzeichnet, dass** im Schritt (A) katalytische Mengen von Metalliodid eingesetzt werden, wobei die Menge des Katalysators zwischen 0,1 und 20 Molprozent, bezogen auf die Verbindungen der allgemeinen Formel (III) liegt.

3. Verfahren gemäß Anspruch 1 oder 2, **dadurch gekennzeichnet, dass** im Schritt (A) 1 bis 2 Moläquivalente hypophosphorige Säure oder Natriumhypophosphit, bezogen auf die Verbindungen der allgemeinen Formel (III), eingesetzt werden.

4. Verfahren gemäß einem der Ansprüche 1 bis 3, **dadurch gekennzeichnet, dass** im Schritt (B) die Metalle Nickel oder Cobalt als Katalysatoren verwendet werden, wobei die Menge des Katalysators 0,01 bis 50 Gewichtsprozent, bezogen auf die Verbindungen der allgemeinen Formel (IV), beträgt.

5. Verfahren gemäß einem der Ansprüche 1 bis 4, wobei die Temperatur im Schritt (B) zwischen 20° und 150°C beträgt.

6. Verfahren gemäß einem der Ansprüche 1 bis 5, wobei die Temperatur im Schritt (A) zwischen 0° und 150°C beträgt.

7. Verfahren gemäß einem der Ansprüche 1 bis 6, **dadurch gekennzeichnet, dass** Verfahrensschritt (A) in einem organischen Lösungsmittel in Gegenwart von Wasser durchgeführt wird.

8. Verfahren gemäß Anspruch 7, **dadurch gekennzeichnet, dass** hypophosphorige Säure oder Natriumhypophosphit NaH₂PO₂ in Gegenwart von Wasser eingesetzt wird.

9. Verfahren gemäß Anspruch 7 oder 8, **dadurch gekennzeichnet, dass** die Menge an Wasser mindestens 0,15 Gewichtsprozent, bezogen auf die Summe an organischem Lösungsmittel und Wasser, beträgt.

10. Verfahren gemäß einem der Ansprüche 7 bis 9, **dadurch gekennzeichnet, dass** das Wasser durch Natriumhypophosphit in Form seines Hydrates NaH₂PO₂ x H₂O bereitgestellt wird.

11. Verfahren gemäß einem der Ansprüche 7 bis 10, **dadurch gekennzeichnet, dass** die Konzentration der Verbindung der allgemeinen Formel (III) mindestens 0,6 mol/l beträgt.

12. Bis(3-nitrophenyl)disulfide der allgemeinen Formel (IV) 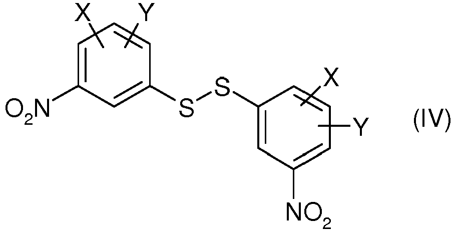 in der die Reste X und Y unabhängig voneinander für Methyl, Ethyl, Methoxy, Ethoxy und Fluor stehen.

13. Verbindungen der Formel (IV) gemäß Anspruch 12, wobei die Verbindungen ausgewählt sind aus
1,1'-Disulfandiylbis(4-fluor-2-methyl-5-nitrobenzol),
1,1'-Disulfandiylbis(2-fluor-4-methyl-5-nitrobenzol),
1,1'-Disulfandiylbis(4-fluor-2-methyl-3-nitrobenzol),
1-Fluor-4-[(4-fluor-2-methyl-5-nitrophenyl)disulfanyl]-3-methyl-2-nitrobenzol,
1-Fluor-4-[(2-fluor-4-methyl-5-nitrophenyl)disulfanyl]-5-methyl-2-nitrobenzol,
1-Fluor-4-[(2-fluor-4-methyl-5-nitrophenyl)disulfanyl]-3-methyl-2-nitrobenzol.

14. Bis(3-aminophenyl)-disulfide der Formel (I) 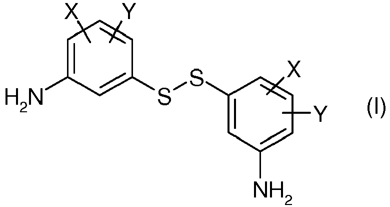 in der die Reste X und Y unabhängig voneinander für Methyl, Ethyl, Methoxy, Ethoxy und Fluor stehen.

15. Verbindungen der Formel (I) gemäß Anspruch 14, wobei die Verbindungen ausgewählt sind aus
3,3'-Disulfandiylbis(6-fluor-4-methylanilin),
3,3'-Disulfandiylbis(4-fluor-6-methylanilin),
3,3'-Disulfandiylbis(2-fluor-6-methylanilin),
3-[(5-Amino-4-fluor-2-methylphenyl)disulfanyl]-6-fluor-2-methylanilin,
5-[(5-Amino-2-fluor-4-methylphenyl)disulfanyl]-2-fluor-4-methylanilin,
3-[(5-Amino-2-fluor-4-methylphenyl)disulfanyl]-6-fluor-2-methylanilin.

16. Bis(3-aminophenyl)-disulfid der Formel (I) 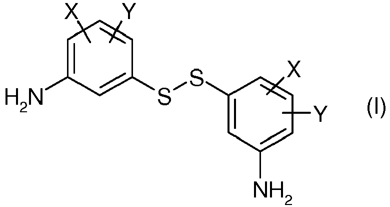 wobei die Verbindung 3,3'-Disulfandiylbis(4,6-dichloranilin) ist.

17. 3-Aminothiole der allgemeinen Formel (II) 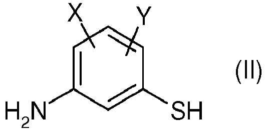 in der die Reste X und Y unabhängig voneinander für Wasserstoff, Methyl, Ethyl, Methoxy, Ethoxy, Fluor und Chlor stehen, wobei die Verbindungen ausgewählt sind aus
5-Amino-2,4-dichlorbenzolthiol,
3-Amino-2-fluor-4-methylbenzolthiol.

## Claims

1. Method for preparing bis(3-aminophenyl) disulphides of the general formula (I) and/or 3-aminothiols of the general formula (II) 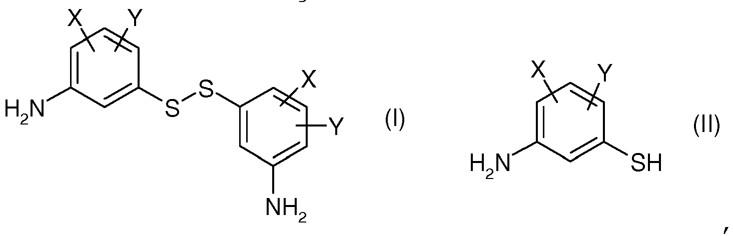 where the residues X and Y are each independently hydrogen, linear or branched (C₁-C₄)-alkyl, linear or branched (C₁-C₄)-alkoxy, halogen or amino;
**characterized in that**
(A) 3-nitrophenylsulphonyl chlorides of the general formula (III) 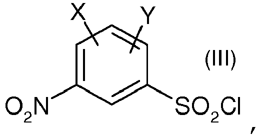 where the residues X and Y have the meanings stated above,
are reduced with catalytic amounts of iodide in the presence of hypophosphorous acid (H₃PO₂) or salts thereof to give bis(3-nitrophenyl) disulphides of the general formula (IV) 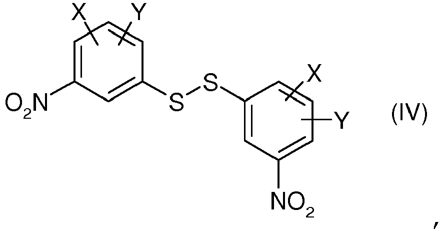 in which the residues X and Y have the meaning stated above,
(B) these compounds of the general formula (IV) are reduced, with hydrogen in the presence of a metal catalyst, to the bis(3-aminophenyl) disulphides of the general formula (I) and/or 3-aminothiols of the general formula (II), where nickel or cobalt are used as metals.

2. Method according to Claim 1, **characterized in that** catalytic amounts of metal iodide are used in step (A), where the amount of catalyst is between 0.1 and 20 mol per cent, based on the compounds of the general formula (III).

3. Method according to Claim 1 or 2, **characterized in that** 1 to 2 mole equivalents of hypophosphorous acid or sodium hypophosphite are used in step (A), based on the compounds of the general formula (III).

4. Method according to any of Claims 1 to 3, **characterized in that** the catalysts used in step (B) are the metals nickel or cobalt, where the amount of catalyst is 0.01 to 50 per cent by weight, based on the compounds of the general formula (IV).

5. Method according to any of Claims 1 to 4, wherein the temperature in step (B) is between 20° and 150°C.

6. Method according to any of Claims 1 to 5, wherein the temperature in step (A) is between 0° and 150°C.

7. Method according to any of Claims 1 to 6, **characterized in that** method step (A) is carried out in an organic solvent in the presence of water.

8. Method according to Claim 7, **characterized in that** hypophosphorous acid or sodium hypophosphite NaH₂PO₂ is used in the presence of water.

9. Method according to Claim 7 or 8, **characterized in that** the amount of water is at least 0.15 per cent by weight, based on the sum of organic solvent and water.

10. Method according to any of Claims 7 to 9, **characterized in that** the water is provided by sodium hypophosphite in the form of its hydrate NaH₂PO₂ x H₂O.

11. Method according to any of Claims 7 to 10, **characterized in that** the concentration of the compound of the general formula (III) is at least 0.6 mol/l.

12. Bis(3-nitrophenyl) disulphides of the general formula (IV) 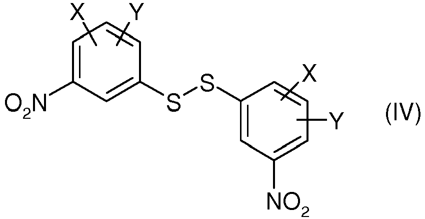 in which the residues X and Y are each independently methyl, ethyl, methoxy, ethoxy and fluorine.

13. Compounds of the formula (IV) according to Claim 12, wherein the compounds are selected from
1,1'-disulphanediylbis(4-fluoro-2-methyl-5-nitrobenzene),
1,1'-disulphanediylbis(2-fluoro-4-methyl-5-nitrobenzene),
1,1'-disulphanediylbis(4-fluoro-2-methyl-3-nitrobenzene),
1-fluoro-4-[(4-fluoro-2-methyl-5-nitrophenyl)disulphanyl]-3-methyl-2-nitrobenzene,
1-fluoro-4-[(2-fluoro-4-methyl-5-nitrophenyl)disulphanyl]-5-methyl-2-nitrobenzene,
1-fluoro-4-[(2-fluoro-4-methyl-5-nitrophenyl)disulphanyl]-3-methyl-2-nitrobenzene.

14. Bis(3-aminophenyl) disulphides of the formula (I) 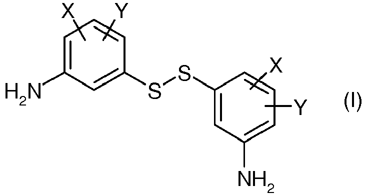 in which the residues X and Y are each independently methyl, ethyl, methoxy, ethoxy and fluorine.

15. Compounds of the formula (I) according to Claim 14, wherein the compounds are selected from
3,3'-disulphanediylbis(6-fluoro-4-methylaniline),
3,3'-disulphanediylbis(4-fluoro-6-methylaniline),
3,3'-disulphanediylbis(2-fluoro-6-methylaniline),
3-[(5-amino-4-fluoro-2-methylphenyl)disulphanyl]-6-fluoro-2-methylaniline,
5-[(5-amino-2-fluoro-4-methylphenyl)disulphanyl]-2-fluoro-4-methylaniline,
3-[(5-amino-2-fluoro-4-methylphenyl)disulphanyl]-6-fluoro-2-methylaniline.

16. Bis(3-aminophenyl) disulphide of the formula (I) 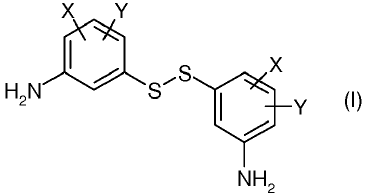 wherein the compound is 3,3'-disulphanediylbis(4,6-dichloroaniline).

17. 3-Aminothiols of the general formula (II) 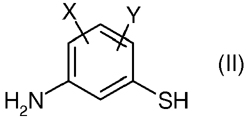 in which the residues X and Y are each independently hydrogen, methyl, ethyl, methoxy, ethoxy, fluorine and chlorine, wherein the compounds are selected from
5-amino-2,4-dichlorobenzenethiol,
3-amino-2-fluoro-4-methylbenzenethiol.

## Revendications

1. Procédé de fabrication de disulfures de bis(3-aminophényle) de formule générale (I) et/ou de 3-aminothiols de formule générale (II) 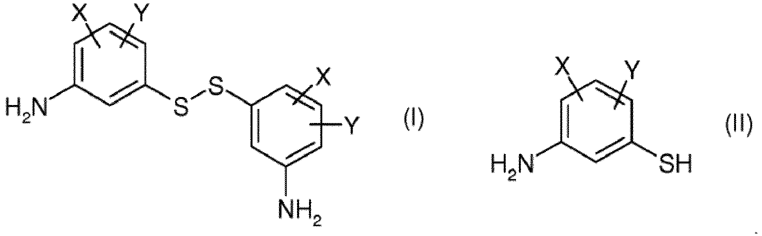 dans lesquelles les radicaux X et Y représentent indépendamment l'un de l'autre l'hydrogène, un alkyle en (C₁-C₄) linéaire ou ramifié, un alcoxy en (C₁-C₄) linéaire ou ramifié, un halogène ou un amino ; **caractérisé en ce que**
(A) des sulfochlorures de 3-nitrophényle de formule générale (III) 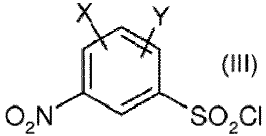 dans laquelle les radicaux X et Y ont les significations indiquées précédemment,
sont réduits avec des quantités catalytiques d'iodure en présence d'acide hypophosphoreux (H₃PO₂) ou ses sels pour former des disulfures de bis(3-nitrophényle) de formule générale (IV) 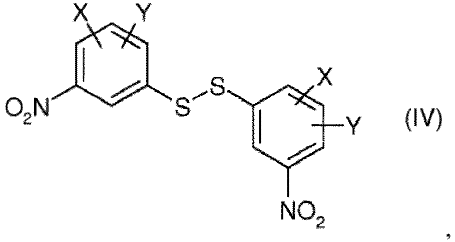 dans laquelle les radicaux X et Y ont la signification indiquée précédemment,
(B) ces composés de formule générale (IV) sont réduits avec de l'hydrogène en présence d'un catalyseur métallique pour former les disulfures de bis(3-aminophényle) de formule générale (I) et/ou les 3-aminothiols de formule générale (II), le nickel ou le cobalt étant utilisé en tant que métal.

2. Procédé selon la revendication 1, **caractérisé en ce qu'**à l'étape (A), des quantités catalytiques d'iodure métallique sont utilisées, la quantité du catalyseur étant comprise entre 0,1 et 20 pour cent en moles, par rapport aux composés de formule générale (III).

3. Procédé selon la revendication 1 ou 2, **caractérisé en ce qu'**à l'étape (A), 1 à 2 équivalents molaires d'acide hypophosphoreux ou d'hypophosphite de sodium sont utilisés, par rapport aux composés de formule générale (III).

4. Procédé selon l'une quelconque des revendications 1 à 3, **caractérisé en ce qu'**à l'étape (B), le métal nickel ou cobalt est utilisé en tant que catalyseur, la quantité du catalyseur étant de 0,01 à 50 pour cent en poids, par rapport aux composés de formule générale (IV) .

5. Procédé selon l'une quelconque des revendications 1 à 4, dans lequel la température à l'étape (B) est comprise entre 20 et 150 °C.

6. Procédé selon l'une quelconque des revendications 1 à 5, dans lequel la température à l'étape (A) est comprise entre 0 et 150 °C.

7. Procédé selon l'une quelconque des revendications 1 à 6, **caractérisé en ce que** l'étape de procédé (A) est réalisée dans un solvant organique en présence d'eau.

8. Procédé selon la revendication 7, **caractérisé en ce que** l'acide hypophosphoreux ou l'hypophosphite de sodium NaH₂PO₂ est utilisé en présence d'eau.

9. Procédé selon la revendication 7 ou 8, **caractérisé en ce que** la quantité d'eau est d'au moins 0,15 pour cent en poids, par rapport à la somme du solvant organique et de l'eau.

10. Procédé selon l'une quelconque des revendications 7 à 9, **caractérisé en ce que** l'eau est mise à disposition par l'hypophosphite de sodium sous la forme de son hydrate NaH₂PO₂ x H₂O.

11. Procédé selon l'une quelconque des revendications 7 à 10, **caractérisé en ce que** la concentration du composé de formule générale (III) est d'au moins 0,6 mol/l.

12. Disulfures de bis(3-nitrophényle) de formule (IV) 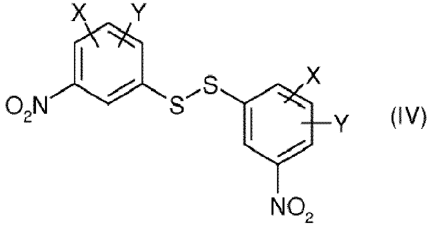 dans laquelle les radicaux X et Y représentent indépendamment l'un de l'autre méthyle, éthyle, méthoxy, éthoxy et fluor.

13. Composés de formule (IV) selon la revendication 12, les composés étant choisis parmi :
le 1,1'-disulfanediylbis(4-fluoro-2-méthyl-5-nitrobenzène),
le 1,1'-disulfanediylbis(2-fluoro-4-méthyl-5-nitrobenzène),
le 1,1'-disulfanediylbis(4-fluoro-2-méthyl-3-nitrobenzène),
le 1-fluoro-4-[(4-fluoro-2-méthyl-5-nitrophényl)disulfanyl]-3-méthyl-2-nitrobenzène,
le 1-fluoro-4-[(2-fluoro-4-méthyl-5-nitrophényl)disulfanyl]-5-méthyl-2-nitrobenzène,
le 1-fluoro-4-[(2-fluoro-4-méthyl-5-nitrophényl)disulfanyl]-3-méthyl-2-nitrobenzène.

14. Disulfures de bis(3-aminophényle) de formule (I) 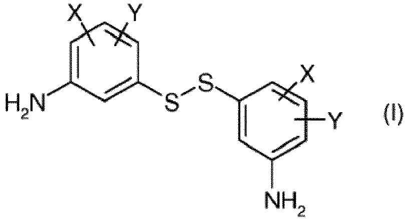 dans laquelle les radicaux X et Y représentent indépendamment l'un de l'autre méthyle, éthyle, méthoxy, éthoxy et fluor.

15. Composés de formule (I) selon la revendication 14, les composés étant choisis parmi :
la 3,3'-disulfanediylbis(6-fluoro-4-méthylaniline),
la 3,3'-disulfanediylbis(4-fluoro-6-méthylaniline),
la 3,3'-disulfanediylbis(2-fluoro-6-méthylaniline),
la 3-[(5-amino-4-fluoro-2-méthylphényl)disulfanyl]-6-fluoro-2-méthylaniline,
la 5-[(5-amino-2-fluoro-4-méthylphényl)disulfanyl]-2-fluoro-4-méthylaniline,
la 3-[(5-amino-2-fluoro-4-méthylphényl)disulfanyl]-6-fluoro-2-méthylaniline.

16. Disulfure de bis(3-aminophényle) de formule (I) 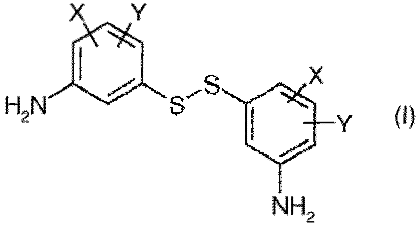 le composé étant la 3,3'-disulfanediylbis(4,6-dichloroaniline).

17. 3-aminothiols de formule générale (II) 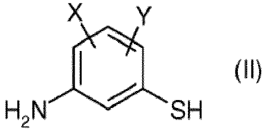 dans laquelle les radicaux X et Y sont choisis indépendamment l'un de l'autre parmi hydrogène, méthyle, éthyle, méthoxy, éthoxy, fluor et chlore, les composés étant choisis parmi :
le 5-amino-2,4-dichlorobenzène-thiol,
le 3-amino-2-fluoro-4-méthylbenzène-thiol.
